(19) Europäisches Patentamt / European Patent Office / Office européen des brevets

(11) **EP 2 882 863 B1**

(12) **EUROPEAN PATENT SPECIFICATION**

(45) Date of publication and mention
of the grant of the patent:
**14.07.2021 Bulletin 2021/28**

(21) Application number: **13829249.5**

(22) Date of filing: **12.08.2013**

(51) Int Cl.:
**C12Q 1/32** (2006.01)     **A61B 6/03** (2006.01)

(86) International application number:
**PCT/US2013/054551**

(87) International publication number:
**WO 2014/028392 (20.02.2014 Gazette 2014/08)**

(54) **SYSTEM AND METHOD FOR QUANTITATIVE MAPPING OF MITOCONDRIAL COMPLEX 1**

SYSTEM UND VERFAHREN ZUR QUANTITATIVEN ABBILDUNG DES MITOCHONDRIEN-KOMPLEXES 1

SYSTÈME ET MÉTHODE DE CARTOGRAPHIE QUANTITATIVE DU COMPLEXE MITOCHONDRIAL 1

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.08.2012 US 201261682601 P**
**14.03.2013 US 201361783468 P**

(43) Date of publication of application:
**17.06.2015 Bulletin 2015/25**

(73) Proprietor: **The General Hospital Corporation Boston, MA 02114 (US)**

(72) Inventors:
• **EL FAKHRI, Georges**
**Brookline, MA 02446 (US)**
• **ALPERT, Nathaniel**
**Boston, MA 02114 (US)**

(74) Representative: **Carpmaels & Ransford LLP**
**One Southampton Row**
**London WC1B 5HA (GB)**

(56) References cited:
WO-A1-90/15575     RU-C1- 2 266 052
US-A1- 2008 009 010     US-A1- 2012 197 619

• **ALPERT NATHANIEL ET AL: "Single-scan rest/stress imagingF-labeled flow tracers", MEDICAL PHYSICS, AIP, MELVILLE, NY, US, vol. 39, no. 11, 1 November 2012 (2012-11-01), pages 6609-6620, XP012160692, ISSN: 0094-2405, DOI: 10.1118/1.4754585 [retrieved on 2012-10-11]**
• **CHRISTOPH RISCHPLER ET AL: "Advances in PET myocardial perfusion imaging: F-18 labeled tracers", ANNALS OF NUCLEAR MEDICINE, SPRINGER JAPAN, JAPAN, vol. 26, no. 1, 9 November 2011 (2011-11-09), pages 1-6, XP035001759, ISSN: 1864-6433, DOI: 10.1007/S12149-011-0552-5**
• **MARCELO F. DI CARLI ET AL.: 'Cardiac PET/CT for the Evaluation of Known or Suspected Coronary Artery Disease' RADIOGRAPHICS vol. 31, no. 5, 2011, pages 1239 - 1251, XP055255997**
• **None**

EP 2 882 863 B1

**Description**

BACKGROUND OF THE INVENTION

Known as the main site for energy production, mitochondria carry out oxidative phosphorylation as the last step for generating adenosine triphosphate (ATP) from the reduced form of nicotinamide adenine dinucleotide (NADH), via a chain reaction of electron transfer. Mitochondrial complex I (MC-I), also known as NADH dehydrogenase or NADH:ubiquinone oxidoreductase, is one of four membrane bound enzymes of the respiratory chain in mitochondria and the first enzyme catalyzing oxidative phosphorylation. There are three energy-transducing enzymes in the electron transport chain: (i) NADH:ubiquinone oxidoreductase (complex I), (ii) Coenzyme Q - cytochrome C reductase (complex III), and (iii) cytochrome C oxidase (complex IV). Complex I is the largest and most complicated enzyme of the electron transport chain. The reaction of complex I is:

$$NADH + H^+ + CoQ + 4H^+_{in} \rightarrow NAD^+ + CoQH_2 + 4H^+_{out}$$

In addition to its key role as initiator of oxidative phosphorylation, MC-I is also regarded as the main site of generation of reactive oxygen species which are harmful to cells.

Genetic disorders of the mitochondria are the most frequent cause of metabolic error, affecting one in five thousand newborns, and deficiency of MC-I is the most common reason. The MC-I defects are thought to be the cause of many diseases, either through the reduced oxidative phosphorylation or by the cellular damage due to reactive oxygen species. On the other hand, abnormal MC-I function or expression level is found in several diseases as a result of hypoxia or necrosis.

In addition to genetic diseases, reduced MC-I expression has also been linked to various neurological, neuromuscular and psychiatric disorders, including Parkinson's disease, Huntington's disease, bipolar disorders and schizophrenia. Another significant field of application is the quantitation of MC-I expression in myocardial ischemia and infarction, conditions known to cause moderate to severe reductions in MC-I. Hence, MC-I is of fundamental importance affecting every living cell in the human body.

There are currently no clinically established methods for in vivo, non-invasive measurement of the MC-I expression level, or equivalently its tissue density. In vitro assay is the current standard laboratory method for such measurements, requiring biopsy or surgery. However, an in vivo quantitative assay of this enzyme would be clinically desirable. Therefore, there is a need for a non-invasive method for quantitative assay and mapping of MC-I expression for clinical and research purposes.

C. Rischpler et al. (in "Advances in PET myocardial perfusion imaging: F-18 labeled tracers", Annals of Nuclear Medicine, Springer Japan, Japan, vol. 26, no. 1, 9 November 2011, pages 1-6) discloses a method using flurpiridaz F-18, formerly known as F-18 BMS747158-02, and F-18 fluorobenzyltriphenylphosphonium (FBnTP) for PET imaging of the heart.

SUMMARY OF ASPECTS OF THE DISCLOSURE

The present disclosure, and invention of claim 1 and it's embodiments as present in the dependent claims, overcomes the above and other drawbacks by providing a non-invasive method for quantitative assay and mapping of MC-I expression for clinical and research purposes. In one aspect, a system and method have been developed to non-invasively and quantitatively map regional MC-I expression by in vivo measurement with quantitative dynamic PET using an MC-I ligand such as $^{18}$F-Flurpiridaz - a chemical analog of pyridaben (an MC-I inhibitor). In one aspect, the system and method are applicable to the field of cardiac PET. In this field, the present in invention can be used to assess coronary artery disease (CAD). In this regard, embodiments of the present invention can provide quantitative mapping of MC-I expression as well as myocardial blood flow (MBF).

In one aspect, a method is provided for in vivo assay and imaging the expression of mitochondrial complex I (MC-I). The method includes the steps of: (i) administering a PET ligand which binds reversibly to MC-I in the tissues of a living subject; (ii) imaging the subject with a positron emission tomography (PET) imaging system and storing at least one PET image of the subject in a non-transitory computer readable media; and (iii) analyzing the kinetic signature of at least one image with a computer processor that identifies portions of the at least one image that represent MC-I expression levels and storing the identifications within non-transitory computer readable media, wherein the representations of MC-I expression levels is based on a PET imageable binding reaction between the PET ligand and MC-I.

In one aspect, the method further includes processing images with a computer processor that calculates a level of blood flow (BF) within the subject by analyzing the kinetic signature of portions of several images expressing levels of the presence of the PET ligand in the subject. In one example, the BF comprises MBF.

In another aspect, not forming part of the claimed invention nor part of the claimed embodiments of the invention, the method further includes identifying states of ischemia and infarction of the myocardium of the subject by assessing both the identified MC-I expression levels and the calculated levels of myocardial blood flow (MBF). The calculated levels of BF and MC-I expression levels can be calculated based on a kinetic model stored within computer readable media.

In still another aspect, the method further includes identifying states of a metabolic disorder, neurological disorder, neuromuscular disorder, and psychiatric disorder based on the identified interaction between a PET ligand and MC-I.

In a second aspect, a system is provided for imaging the expression of mitochondrial complex I (MC-I). The system includes: a positron emission tomography (PET) imager; a source of PET radiotracer for administration to a subject; and a processor having non-transient computer readable media programmed with instructions to obtain PET images of the subject administered with the radiotracer. The computer readable media is programmed with instructions to process the PET images and identify portions of the PET images that represent MC-I expression levels. Furthermore, the representations of MC-I expression levels are based on PET imageable interactions between the radiotracer and MC-I.

In another aspect, a method is provided for imaging the expression of mitochondrial complex I (MC-I). The method includes the steps of: (i) administering a radiotracer that serves as chemical analog of an MC-I inhibitor; (ii) imaging the subject with a positron emission tomography (PET) imaging system to acquire PET data from the subject; (iii) reconstructing image(s) of the subject from the PET data; (iv) processing the image(s) with a computer processor to identify portions of the image(s) that represent MC-I expression levels; and (v) generating a report including representations of MC-I expression levels based on the image(s) of the subject and the portions of the image(s) that represent MC-I expression levels.

In one aspect, the radiotracer includes $^{18}$F-Flurpiridaz. In another aspect, the method includes processing the image(s) with a computer processor to calculate a level of blood flow (BF) within the subject by analyzing portions of the image(s) of the subject expressing levels of the presence of the radiotracer. In one example, not forming part of the claimed invention nor part of the claimed embodiments of the invention, the report includes an indication of states of ischemia and infarction of the myocardium of the subject correlated to MC-I expression levels and levels of BF.

In another aspect, the report includes a quantification of at least one of MC-I expression activity levels and BF levels. In one example, the method further includes identifying states of a metabolic disorder, neurological disorder, neuromuscular disorder, and psychiatric disorder based on the identified interaction between the radiotracer and MC-I. In yet another aspect, the radiotracer provides a unidirectional extraction fraction that approaches unity at a plurality of physiological flow values.

In yet another aspect, a system is provided for imaging the expression of mitochondrial complex I (MC-I). The system includes: a non-transitive storage medium on which is stored positron emission tomography (PET) image(s) of a subject having received a dose of a radiotracer that serves as chemical analog of an MC-I inhibitor; and a processor having non-transient computer readable media programmed with instructions to cause the processor to access the PET image(s) and process the PET image(s) to identify portions of the PET image(s) that represent MC-I expression levels.

In one aspect, the system further includes a display to provide an image indicating the portions of the PET image(s) that represent MC-I expression levels. In another aspect the processor analyzes the PET image(s) to determine a kinetic signature. In yet another aspect, the processor calculates a level of blood flow (BF) within the subject by analyzing the kinetic signature of portions of the PET image(s) expressing levels of the presence of the PET ligand in the subject. In another aspect, the processor calculates myocardial blood flow (MBF) using the PET image(s) and, in an example not forming part of the claimed invention nor part of the claimed embodiments of the invention, further identifies states of ischemia and infarction of myocardium of the subject by assessing both identified MC-I expression levels and the calculated levels of MBF.

The foregoing and other advantages of the invention will appear from the following description. In the description, reference is made to the accompanying drawings that form a part hereof, and in which there is shown by way of illustration a preferred embodiment in accordance with the invention.

BRIEF DESCRIPTION OF THE DRAWINGS

Fig. 1A-1B is a schematic illustration of MC-I quantitation.
Fig. 1C shows the molecular structure or $^{18}$F-Flupiridaz.
Fig. 2A shows the summed images of the myocardium in the short and long axis.
Fig. 2B is a graph showing a comparison of the input functions derived with GFADS and blood samples (points) and ROI-based input function.
Fig. 3A is a graph showing activity data from a monkey study fit with a 2-compartment, reversible model.
Fig. 3B is a graph showing K1 data from the monkey study with the estimated K1 as a function of study duration.

Fig. 3C is a graph showing BP data from the monkey study with $BP_P$ illustrated to take longer (-60 minutes) for a reliable estimate.

Fig. 4A is a graph showing the kinetics of the tracer uptake in a dynamic rest-stress study.

Fig. 4B shows co-registered rest and stress images of the $^{18}$F-Flurpiridaz in clinical cardiac studies.

Fig. 5 is a graph showing a comparison of estimated $BP_P$ with 60-min and 100-min studies in three types of myocardium using simulation studies.

Fig. 6A shows $^{18}$F-Flurpiridaz PET images from infarcted pig in the short (top row) and long (middle and lower rows) axis views. Arrows indicate site of infarction.

Fig. 6B shows photographs acquired at dissection that confirm the infarction on the anteroseptal wall (left: epicardial view, right: myocardium inverted for endocardial view).

Fig. 6C shows a western blot indicating reduced MC-I expression in partially and completely infarcted tissue samples.

Fig. 6D shows two-tissue compartment model fits to $^{18}$F-Flurpiridaz PET data in healthy (upper panels) and ischemic (lower panels) myocardium. Coupled fits (right) have fewer free variables and exhibit minor degradation in the quality of model fits to data, thereby reducing variance of estimated parameters.

Fig. 7 is a schematic view of an emission tomography system in accordance with the present invention.

Fig. 8 is a flow chart setting forth the steps of an example of a method of using an emission tomography system in accordance with the present invention.

## DETAILED DESCRIPTION OF THE INVENTION

The system and methods described, in one embodiment, contemplate the use of an in vivo assay to measure MC-I expression levels. While specific examples are provided, it is contemplated that a general implementation could include the use of an MC-I targeting molecule that is compatible with one or more of a variety of medical imaging technologies. For example, in one embodiment, a method includes the steps of administering the MC-I targeting molecule $^{18}$F-Flurpiridaz for PET imaging. The MC-I targeting molecule used for PET imaging may be compatible with magnetic resonance imaging (MRI), ultrasound imaging, or any other type of suitable imaging technology.

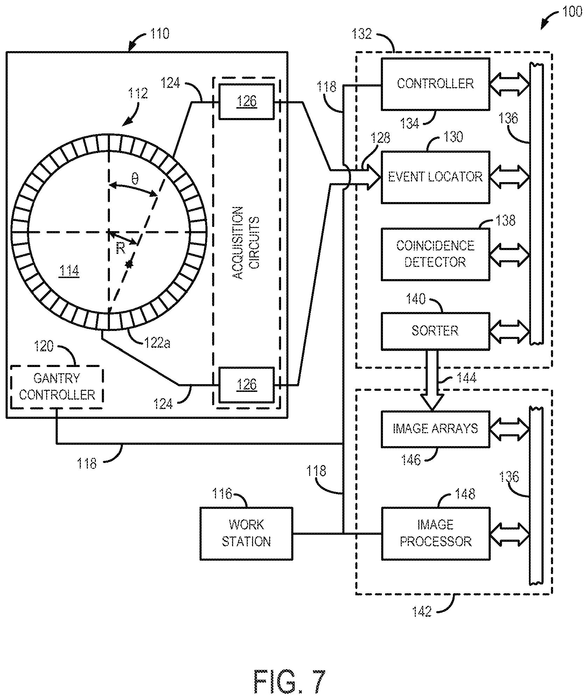

Referring to Fig. 7, a PET system 100 in accordance with the present invention includes an imaging hardware system 110 that includes a detector ring assembly 112 about a central axis, or bore 114. An operator work station 116 including a commercially-available processor running a commercially-available operating system communicates through a communications link 118 with a gantry controller 120 to control operation of the imaging hardware system 110.

The detector ring assembly 112 is formed of a multitude of radiation detector unit 122 that produces a signal responsive to detection of a photon on communications line 124 when an event occurs. A set of acquisition circuits 126 receive the signals and produce signals indicating the event coordinates (x, y) and the total energy associated with the photons that caused the event. These signals are sent through a cable 128 to an event locator circuit 130. Each acquisition circuit 126 also produces an event detection pulse that indicates the exact moment the interaction took place. Other systems utilize sophisticated digital electronics that can also obtain this information regarding the precise instant in which the event occurred from the same signals used to obtain energy and event coordinates.

The event locator circuits 130 in some implementations, form part of a data acquisition processing system 132 that periodically samples the signals produced by the acquisition circuits 126. The data acquisition processing system 132 includes a general controller 134 that controls communications on a backplane bus 136 and on the general communications network 118. The event locator circuits 130 assemble the information regarding each valid event into a set of numbers that indicate precisely when the event took place and the position in which the event was detected. This event data packet is conveyed to a coincidence detector 138 that is also part of the data acquisition processing system 132.

The coincidence detector 138 accepts the event data packets from the event locator circuit 130 and determines if any two of them are in coincidence. Coincidence is determined by a number of factors. First, the time markers in each event data packet must be within a predetermined time window, for example, 0.5 nanoseconds or even down to picoseconds. Second, the locations indicated by the two event data packets must lie on a straight line that passes through the field of view in the scanner bore 114. Events that cannot be paired are discarded from consideration by the coincidence detector 138, but coincident event pairs are located and recorded as a coincidence data packet. Thes coincidence data packets are provided to a sorter 140. The function of the sorter in many traditional PET imaging systems is to receive the coincidence data packets and generate memory addresses from the coincidence data packets for the efficient storage of the coincidence data. In that context, the set of all projection rays that point in the same direction ($\theta$) and pass through the scanner's field of view (FOV) is a complete projection, or "view". The distance (R) between a particular projection ray and the center of the FOV locates that projection ray within the FOV. The sorter 140 counts all of the events that occur on a given projection ray (R, $\theta$) during the scan by sorting out the coincidence data packets that indicate an event at the two detectors lying on this projection ray. The coincidence counts are organized, for example, as a set of two-dimensional arrays, one for each axial image plane, and each having as one of its dimensions the projection angle $\theta$ and the other dimension the distance R. This $\theta$ by R map of the measured events is call a histogram or, more commonly, a sinogram array. It is these sinograms that are processed to reconstruct images that indicate the number of events that took place at each image pixel location during the scan. The sorter 140 counts all events occurring along each projection ray (R, $\theta$) and organizes them into an image data array.

The sorter 140 provides image datasets to an image processing/reconstruction system 142, for example, by way of a communications link 144 to be stored in an image array 146. The image arrays 146 hold the respective datasets for access by an image processor 148 that reconstructs images. The image processing/reconstruction system 142 may communicate with and/or be integrated with the work station 116 or other remote work stations.

Deficiency in mitochondrial complex I (MC-I) is a common feature affecting a wide gamut of genetic, psychiatric and cardiac diseases. A system and method has been that provides a non-invasive approach to measure the in vivo MC-I expression level. In one aspect, the present disclosure utilizes an intravenous injection of a tracer amount of a radionuclide $^{18}$F-Flurpiridaz. However, it is anticipated that other molecules, such as chemical analogs of pyridaben, an MC-I inhibitor, can be used in the implementation of the system and methods described herein, but such use of said other molecules does not form part of the claimed invention. In general, any molecule which gains ready access to the extravascular space and binds selectively and reversibly to the MC-I complex can potentially be used to measure the expression of MC-I. However, binding ligands will have a range of properties which can be optimized for the purpose of MC-I assay. For example, the kinetics of $^8$F-Flurpiridaz binding are relatively slow. A measurement might take an hour or more. In certains aspects, therefore $^{18}$F-Flurpiridaz is suitable for human research. In other aspects, a particular ligand can be useful as a blood flow agent independent of the usefulness of the ligand for an MC-I assay and vice versa. Overall, $^{18}$F-Flurpiridaz is an existing example ligand that has shown the ability to reversibly bind to MC-I and one may expect that many agents can be made which will have suitable properties for the implementation of the present invention. In addition, it is likely that other mechanisms can be found to design molecules that can be used to measure MC-I binding.

$^{18}$F-Flurpiridaz is a PET tracer currently in clinical trials to assess myocardial blood flow (MBF). The present invention recognizes that Flurpiridaz has a number of important kinetic properties including a unidirectional extraction fraction that is close to 1 at all physiological flow values, and the trait of being a chemical analog of pyridaben, an MC-I inhibitor. Flurpiridaz, currently under Phase III investigation, is regarded as a promising cardiac imaging agent, but its potential for imaging/measuring MC-I expression has not heretofore been discovered or harnessed.

In developing the present invention, experiments were carried out that show that the first few minutes of $^{18}$F-Flurpiridaz concentration history is determined solely by blood flow; whereas, its later time course is determined by reversible binding to MC-I, permitting the quantitative mapping of its binding potential with PET imaging and kinetic modeling. Thus, it was determined that, in addition to its use as a flow tracer, a noninvasive, in vivo assay of MC-I expression could be developed. In one aspect, within the same imaging session and with the same injected dose of tracer, both the perfusion and the MC-I expression level can be quantified. In examples not forming part of the claimed method, nor claimed embodiments, the ability to non-invasively quantify MC-I expression levels can potentially be translated to the diagnosis of various diseases related to mitochondrial abnormalities or the study of the pathological role of mitochondria in these diseases. In addition (in examples not forming part of the claimed method nor claimed embodiments), measuring both perfusion and MC-I in the same study may extend the ability to diagnose hibernating myocardium in patients with chronic myocardial ischemia.

$^{18}$F-Flurpiridaz PET dynamic studies provide accurate and precise quantitation of the MC-I expression level using kinetic modeling analysis. The conditions under which the binding potential of the MC-I can be computed precisely with PET studies can be determined by a first set of experiments. Using this information, PET acquisition can be optimized to achieve an optimal experimental design. A previously developed porcine model of chronic myocardial ischemia can be used to vary MC-I and myocardial blood flow levels and validate PET measurements by direct comparison to in vitro MC-I assays and microspheres, respectively.

In one aspect of the disclosure, the dual functions of $^{18}$F-Flurpiridaz for quantitatively mapping both the MBF and the MC-I activity can be ascertained. Currently, most work done for $^{18}$F-Flurpiridaz focuses on the semi-quantitative imaging of MBF. In one aspect, the approach presented herein further extends the use of $^{18}$F-Flurpiridaz to simultaneous measurement of perfusion and MC-I within a single dynamic scan session. Kinetic modeling and graphical analysis can be used as the data analysis approach and the technical details for such analysis can be addressed, including the input function extraction and determination of the required study duration. In the field of nuclear medicine, imaging techniques often involve taking a single picture of an activity distribution. In certain aspects of the disclosure, the methods described herein include the acquisition of multiple images spaced in time, followed by one or more calculations based on a model of the kinetic behavior of the MC-I ligand. In one aspect, the model and calculation are applicable to all MC-I ligands/tracers.

In another emobodiment, MC-I expression level measured with $^{18}$F-Flurpiridaz can be validated in a porcine model by standard in vitro assays. In the animal model, MC-I expression levels in different myocardial territories can be altered by different degrees of hypoxia; therefore a spectrum of the MC-I expression level will be created in the heart. Through in vitro analysis, MC-I expression can be assayed in those diseased regions as well as in the viable myocardium. These measurements made with in vivo $^{18}$F-Flurpiridaz studies can then be evaluated in terms of accuracy and precision with statistical measures and tests. MBF quantitation can be validated using microspheres, which can provide the reference flow in all cardiac territories.

In yet another aspect of the disclosure, not forming part of the claimed invention nor part of the claimed embodiments of the invention, the relationship between the degree of myocardial ischemia and the MC-I expression level can be quantitatively examined. In one aspect of the disclosure, not forming part of the invention as claimed nor claimed embodiments, based on the in vitro assay and histology, myocardial samples can be classified as normal, hibernating and infarcted myocardium. In an aspect not forming part of the invention as claimed nor claimed embodiments, MC-I expression level measured with $^{18}$F-Flurpiridaz PET can be compared and it can be determined if such measurements can be used as a potential tool to differentiate these three types of tissue non-invasively. In another aspect, the discriminant power that can be achieved when discriminating among the three tissue classes using 1) perfusion-only, 2) MC-I only and 3) perfusion and MC-I combined can be assessed.

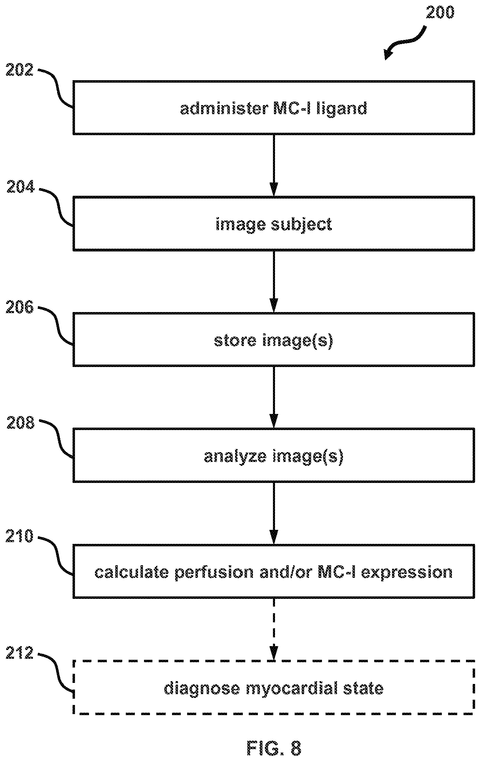

Referring to Fig. 8, a flow chart is provided that sets forth the steps of an example of a method 200 of using an emission tomography system in accordance with the present invention. In a first step 202 of method 200, a dose suitable tracer molecule for binding MC-I (MC-I ligand) is administered. One example of an MC-I ligand is the PET tracer $^{18}$F-Flurpiridaz. The dose is, in one aspect, administered to a subject such as a human patient. In a next step 204 of the method 200, the subject is imaged using an emission tomography system such as a PET system. The subject is imaged for a period of time based on the kinetics of the MC-I ligand. For example, when the MC-I ligand is $^{18}$F-Flurpiridaz, the subject can be imaged within the first few minutes of the administering step if the goal is to calculate perfusion. However, if it is desirable to calculate MC-I expression, then the subject can be imaged for a longer overall time frame or beginning at a later time after the step of administering.

In a next step 206 of method 200, the one or more images of the subject acquired with the emission tomography system can be stored in a non-transitory computer readable media. Then, in a step 208 of method 200, the one or more images can be analyzed, for example, with a computer processor. Following analysis, in step 210, one or more calculations can be made. The calculations can include quantification of subject perfusion and MC-I expression levels. Furthermore, in optional step 212 of method 200, not forming part of the claimed invention nor its claimed embodiments, the subject myocardium can be diagnosed as hibernating myocardium, healthy myocardium and/or ischemic myocardium.

With reference to the above method 200, it is possible that implement the steps in the order shown or in any order suitable to the successful execution of the method 200. For an example not forming part of the claimed invention, imaging of the subject in step 204 may be carried out prior to and concurrent with administration of the MC-I ligand in step 202. In another example, the step 208 of image analysis can be intermittent with calculations in step 210. While a number of examples have been described, other useful combinations of the steps of method 200 are both likely and anticipated.

In examples not forming part of the claimed invention, nor part of the claimed embodiments, the ability to non-invasively quantify MC-I expression levels can be translated to the diagnosis of various diseases related to mitochondrial abnormalities or the study of the pathological role of mitochondria in these diseases. In addition, measuring both perfusion and MC-I in the same study extends, in examples not forming part of the claimed invention, nor part of the claimed embodiments, to the ability to diagnose hibernating myocardium in patients with chronic myocardial ischemia

Currently, there are no established methods for in vivo, non-invasive measurement of the MC-I expression level, or equivalently its tissue density. In vitro assay is the current standard laboratory method for such measurements, requiring biopsy or surgery. By using the tracer $^{18}$F-Flurpiridaz (formerly known as $^{18}$F-BMS7472158-02), the present disclosure, and invention as claimed, provides an imaging approach to quantify the MC-I expression level for clinical and research purposes. $^{18}$F-Flurpiridaz is a pyridaben analog that has been shown to bind to the mitochondria complex I (MC-I). First developed as a myocardial perfusion tracer, it has several attractive features in that regard: high first-pass extraction fraction, long retention in the myocardium, and an $^{18}$F-label which allows commercial distribution to sites without a cyclotron and produces excellent image quality, due to the short positron range. Heretofore, the affinity of the tracer for MC-I have been largely ignored. As an MC-I inhibitor with a high binding affinity, the present invention makes it possible to measure MC-I binding potential and use it to determine the MC-I expression levels. Exemplary applications include coronary artery diseases (CAD) and neuropsychiatric diseases and genetic MC-I deficiencies.

There have been a few reports studying the effect of ischemic injuries, either acute or chronic, on MC-I. In animal models, it has been shown that animals with induced chronic ischemia (which is also thought to be the cause of hibernating myocardium) have reduced MC-I expression levels in the myocardium. The theory behind this phenomenon is that MC-I expression is lowered because 1) the energy demand and perfusion are reduced in these areas so that MC-I expression is down regulated to accommodate the lowered metabolism, and 2) a fraction of myocardial cells in these areas become non-viable or scarred, fibrous tissue due to the ischemic injury. The overall density of mitochondria is reduced and therefore leads to lowered MC-I expression. As a result, observation of significantly reduced MC-I expression in the diseased myocardium creates a possibility for using $^{18}$F-Flurpiridaz PET imaging in such applications. In one aspect, an advantage of $^{18}$F-Flurpiridaz PET is that the perfusion can be measured within the same imaging session in addition to MC-I expression, which may, in examples not forming part of the claimed invention, nor part of the claimed embodiments, lead to more definitive diagnosis of ischemic but viable injury versus infarction.

The link between abnormality of mitochondria functions and various neuropsychiatric diseases has been discussed in recent reports and still remains an active research topic. MC-I deficiency has been shown to play an important role in the dopaminergic neuron damage in Parkinson's disease. Similar MC-I deficiencies have also been found in Huntington's disease, Alzheimer's disease, Down syndrome, schizophrenia, and bipolar disorders. Since the link between MC-I abnormalities and these diseases was discovered in recent years, this is relatively a new field that can potentially benefit from the system and methods described herein. Except by biopsy, it is not currently possible to assess regional abnormalities in MC-I expression. In one aspect of the disclosure, (not forming part of the claimed invention, nor part of the claimed embodiments), a PET imaging technique for regional MC-I assay ($^{18}$F-Flurpiridaz crosses the brain blood barrier) opens up opportunities to evaluate new therapeutic agents and will help diagnose and possibly advance treatment management for MC-I deficiency diseases, such as Leigh syndrome, renal tubular acidosis and cardiomyopathy.

The described system and methods have scientific and clinical implications by providing an in vivo and non-invasive technique to measure the MC-I expression level. Because PET is a well-established imaging technique for clinical diagnosis and research, the described methods can, in aspect have great potential for translational studies and clinical applications. In another aspect, in addition to the MC-I expression, the same $^{18}$F-Flurpiridaz study and kinetic modeling analysis also can be used to quantify tissue perfusion. This feature is particularly attractive for cardiac applications, in which perfusion and metabolism information can often increase (in examples not forming part of the claimed invention, nor part of the claimed embodiments) the diagnostic sensitivity when they are used together.

EXAMPLES

**EXAMPLE 1**

*Methods for quantitating MC-I expression level from a single $^{18}$F-Flurpiridaz PET:*
MC-I expression level can be defined using Equation 1:

$$\lambda_{MC-I} = \alpha \cdot B_{\max} \quad \text{(Eq. 1)}$$

where $\alpha$ is a proportionality constant and $B_{max}$ is the local concentration of MC-I in tissue. Assuming that the affinity of Flurpiridaz for MC-I is not altered in disease or pathological condition, Eq. 1 can be written as

$$\lambda_{MC-I} = \alpha' \cdot BP \quad \text{(Eq. 2)}$$

where $\alpha' = \alpha \cdot K_D$ is a constant of proportionality, $1/K_D$ is the affinity of the ligand (Flurpiridaz) for MC-I, and BP denotes the binding potential. Thus, it was hypothesized that Flurpiridaz binding potential could serve as a surrogate endpoint for the expression level of MC-I.

For the purpose of an initial validation study and proof of principle, a chronic porcine ischemia model as chosen. This model was shown to provide normal, decreased (ischemic), and very low (infarcted) activities of MC-I, , as described by Page B, Young R, Iyer V, et al. Persistent regional downregulation in mitochondrial enzymes and upregulation of stress proteins in swine with chronic hibernating myocardium. Circ Res. Jan 4 2008;102(1):103-112. The porcine model has additional advantages for PET studies, including a model size similar to man and suitability for quantitative dynamic imaging.

**EXAMPLE 2**

*Experimental design:*

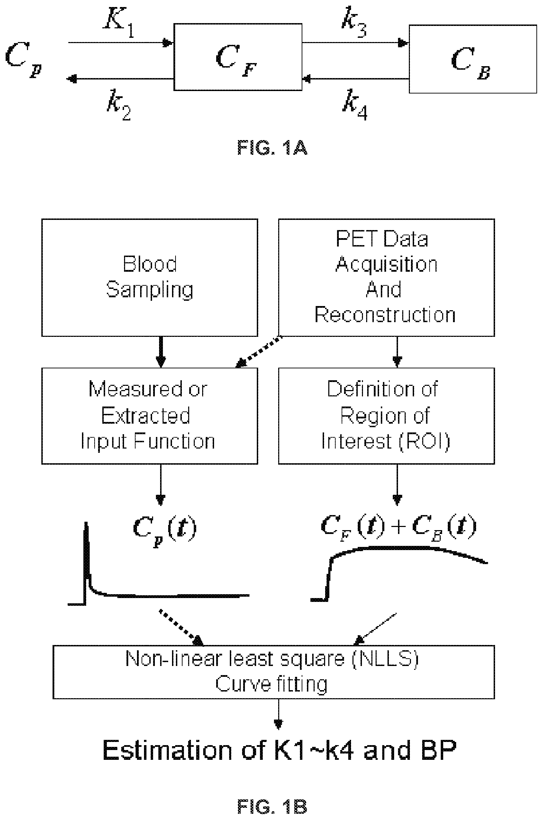

Measurement of myocardial blood flow and binding potential with $^{18}$F-Flurpiridaz can be approached with standard methods that are well understood and available in our laboratory. We use a four parameter ($K_1$, $k_2$, $k_3$, $k_4$), two tissue compartment model (Figs. 1A-1B) to describe Flurpiridaz kinetics. In this model the tracer can be in one of two states, either free, as the injected molecule, or bound to a receptor. In the case of $^{18}$F-Flurpiridaz, previous studies have shown that it binds reversibly to mitochondrial complex I and this is supported by the data presented herein (see Example 3). Referring to Figs. 1A-1B, the product of plasma concentration ($C_p$) and transport rate $K_1$ is the force driving the tracer from capillary plasma to the free space (i.e., both free and nonspecifically bound). Conceptually, $C_F$ represents free flurpiridaz and $C_B$ represents Flurpiridaz binding to the MC-I complex. Corrections (not shown) are included for spillover

of activity.

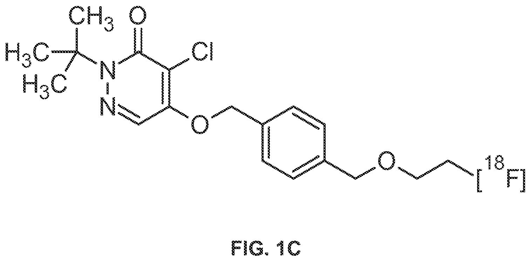

Symbolically, $K_1$ is the plasma-to-tissue transport rate, FE, the product of flow and the unidirectional capillary extraction fraction, E. $k_2$ is a rate constant summarizing the egress from tissue to plasma. The rate constants $k_3$ ($k_{on}*B'_{max}$) and $k_4$ ($k_{off}$) provide a summary description of binding and release of tracer. This model, with nonlinear least squares, was used to fit 100 minutes of data obtained in a study of binding in the heart of a cynomolgus monkey (see Example 3). It is anticipated that a similar approach will be adequate for validation studies in pigs. Graphical methods can also be studied and applied for determination of BP, as they may be more appropriate for quantitative parametric images and future translation to human studies. For example, consider: Logan J, Fowler JS, Volkow ND, et al. Graphical analysis of reversible radioligand binding from time-activity measurements applied to [N-11C-methyl]-(-)-cocaine PET studies in human subjects. J Cereb Blood Flow Metab. Sep 1990;10(5):740-747 and Zhou Y, Ye W, Brasic JR, Crabb AH, Hilton J, Wong DF. A consistent and efficient graphical analysis method to improve the quantification of reversible tracer binding in radioligand receptor dynamic PET studies. Neuroimage. Feb 1 2009;44(3):661-670. Software programs for both approaches have been developed. Realistic Monte Carlo simulation and sensitivity analysis can be used to determine the precision obtained with a given dose of $^{18}$F-Flurpiridaz and the experimental duration of the study. The structure of $^{18}$F-Flurpiridaz is detailed in Fig. 1C.

In some aspects the model is further developed. The modeling approach is based upon the two-tissue compartment model that describes the uptake and retention of a radiotracer that diffusively exchanges between the blood and tissue, and binds reversibly to a specific target site (FIG. 1A-1B). In this framework the concentration of radiotracer is considered in three distinct states: in the plasma ($C_P$) or one of two tissue states, freely suspended in the tissue fluids ($C_F$) or specifically bound to the target site ($C_B$). Four parameters ($K_1$, $k_2$, $k_3$, $k_4$) detail the physiological mechanisms underlying uptake and binding. $K_1$ and $k_2$ pertain to the rates of tracer influx (proportional to blood flow) from the vasculature and efflux back out of the tissue, respectively. The ratio $K_1/k_2$ is the partition coefficient $V_{ND}$. which reflects nonspecific uptake based on properties of the tissue that are not expected to vary regionally. The rate at which tracer binds to the target site is related by $k_3$. The bimolecular nature of associative binding makes $k_3$ a compound parameter, the product of $k_{on}$ (an intrinsic binding rate characteristic to the ligand and target site) and $B_{max}$ (the density of the binding sites). For reversibly binding interactions such as between $^{18}$F-Flurpiridaz and MC-I, the return of specifically bound tracer to the free state is portrayed by $k_4$. Here $k_4$ is synonymous with the intrinsic binding dissociation constant $k_{off}$, which is not expected to differ between regions. In our cardiac application, two additional parameters ($f_{LV}$, $f_{RV}$) account for signal contamination from blood pools in the adjacent left and right ventricles. Combinations of individual rate constants yield macroparameters that are robust and informative with regard to radiotracer binding at the target site. These macroparameters include total volume of distribution ($V_T=[K_1/k_2][1 +k_3/k_4]$, the equilibrium ratio of tracer concentration in tissue vs. plasma) and several variants of binding potential (BP, proportional to the target density). An excellent review of these outcomes is provided in Wu et al., The Journal of Nuclear Medicine. 1998;39:117-425.

The two-tissue compartmental approach is sometimes unable to provide unique or precise estimates of individual rate constants or macroparameters, especially for radiotracers with slow kinetics such as $^{18}$F-Flurpiridaz. In these instances, graphical transformations of the model may yield more robust, though sometimes biased, characterizations of tracer uptake and binding. In addition, these linearized methods are computationally efficient and may facilitate pixel-by-pixel analysis for the generation of parametric images. Therefore, it can be beneficial to study these techniques and assess the bias/precision tradeoff. Another approach to improve the stability of outcome parameters and still remain within the compartmental model framework is to analyze multiple regions simultaneously in a "grand fit" that couples parameters which are common or otherwise related between the different regions. As noted above, the ratio $K_1/k_2$ and $k_4$ are expected to have little or no physiological variation between regions and will be considered for coupled fitting. This approach can be characterized with regard to the validity of the assumption of spatial uniformity and the compromise between accuracy and precision of the outcome parameters.

*Plasma input function:*

All methods for estimation of blood flow and BP can benefit from measurement of the input function. The choice of the porcine model of myocardial ischemia is advantageous for PET imaging in that the concentration history for myocardium, left ventricle and right ventricle can be obtained simultaneously and noninvasively. Concentration curves for left and right ventricle by generalized factor analysis (GFADS) of the dynamic PET data can also be obtained as described: El Fakhri G, Kardan A, Sitek A, et al. Reproducibility and accuracy of quantitative myocardial blood flow assessment with (82)Rb PET: comparison with (13)N-ammonia PET. J Nucl Med. Jul 2009;50(7):1062-1071 and El Fakhri G, Sitek A, Guerin B, Kijewski MF, Di Carli MF, Moore SC. Quantitative dynamic cardiac 82Rb PET using generalized factor and compartment analyses. J Nucl Med. Aug 2005;46(8):1264-1271.

In one aspect, GFADS is valuable in the implementation of the system and methods described herein as the GFADS input function is robust to noise and minimally affected by spillover. Supplemental blood samples can be drawn to validate the PET-arterial concentrations and to determine the activity ratio for $^{18}$F-Flurpiridaz in plasma versus whole blood and to characterize labeled metabolites: Samples can be weighed, counted, then spun down to measure whole blood and plasma activity concentrations. Hematocrit and standard blood-gas measurements can also be performed. The free fraction of Flurpiridaz can be determined for each pig by ultrafiltration. Plasma metabolite analysis can be performed by HPLC. If the plasma free fraction is constant across pigs, BP can be computed as $BP_p$; Alternatively, BP can be computed as $BP_f$.

*Surgical preparation of animals to induce chronic ischemia in the porcine myocardium:*

Animal protocols can be useful for implementation of the system and methods described herein. In one aspect, domestic swine of either sex can be used for in the implementation of the model. Endotracheal intubation and mechanical ventilation can be performed under general anesthesia. Prior to intubation and surgery, pigs can be pre-medicated with the intravenous administration of thiamylal. In one aspect, anesthesia can be maintained with Halothane (0.5-1.5 %) and a mixture of nitrous oxide (60%) and oxygen (40%). Central hemodynamics (e.g., arterial pH, pCO2, pO2) and ECG can be surveyed and maintained within the physiological range. In each pig, it is possible to induce both a partial (causing hibernating myocardium) and a complete stenosis (causing infarcted myocardium). For the hibernating myocardium, imaging can take place after two to four weeks to allow time for development. However, longer delays can be necessary to achieve hibernating status, in which case, the animal protocol can be adjusted accordingly.

To induce the hibernating myocardium, a partially occlusive stenosis can be created by incomplete ligation of the left anterior descending (LAD) coronary artery. Thoracotomy can be performed at the level of the left fourth intercostals space through a 10-15 cm excision. The proximal third of the LAD artery can be partially sutured to induce chronic low level ischemia in the LAD territory. A 25-gauge cannula can be placed in the LAD artery distal to the occlusion to monitor arterial pressure. Myocardial blood flow can be decreased to 40% in the LAD territory.

To induce the infarcted myocardium, a complete stenosis can be created by complete ligation in the distal left circumflex (LCX) coronary artery. Similar procedures can be used as above. ECG and hemodynamics can be carefully monitored after the occlusion to ensure the survival of the animal.

After surgery, the wound can be closed and sutured. The animal can receive a fentanyl patch for pain management applied on the day of surgery and continued for 72 hours. In one aspect, after the immediate postoperative period, the animal is observed daily. In another aspect, the animal is observer at least two times daily and additional observation can be valuable. After a period of time, for example, two to four weeks after the occlusion, pigs can be imaged with $^{18}$F-Flurpiridaz in order to confirm ischemia. If the scan reveals a dysfunctional area within the LAD territory, the hibernating myocardium is established and it can be determined to proceed with actual $^{18}$F-Flurpiridaz PET. Otherwise, the procedure can be delayed for an additional period of time (e.g., one week) and a PET scan can be repeated to evaluate the myocardium until the hibernating status can be confirmed. Once the hibernating state is established, pigs can be imaged on the second day or within seven days to allow the decay of $^{18}$F-Flurpiridaz.

*Imaging protocol for $^{18}$F-Flurpiridaz PET animal studies:*

All PET studies can be performed using any suitable analysis method. For example, one method of analysis includes positron emission tomography and computed tomography (PET-CT). Before the PET study, a thoracotomy can be performed in the left fifth intercostal space to insert a catheter into the left atrium for injection of the microspheres during the PET studies. The animal can undergo a dynamic PET study for 120 minutes with a 10 mCi injected dose of $^{18}$F-Flurpiridaz. However, the time frames for the study and the dosing amount of $^{18}$F-Flurpiridaz can be varied depending on the desired outcome of the study. Radioactive microspheres can be injected via a catheter into the left atrium, to provide a reference flow measurement. During the PET study, arterial blood samples can be taken for measurement of the reference input function (see Example 1 - plasma input function). At the end of the PET study the pig can be sacrificed. The excised hearts can first be infused with Monastral blue through the RCA to stain the intact myocardium and then trimmed of the atria and right ventricle. The excised heart can be re-oriented into the major vertical long axis - horizontal long axis referential to perform short axis slicing of the myocardium into slices of equal thickness (e.g., 8 slices). Each slice can be further subdivided into sections large enough to provide good precision for in vitro and microsphere assay - for example, about 25 sections. Each section can be stained with Triphenyl Tetrazolium Chloride and characterized for its ischemic status. Tissue samples can be counted in a well counter for measurement of microsphere flow. Following that, the tissue samples can be used in MC-I activity assay. ROI analysis can be used to extract concentration histories for compartment analysis. ROIs can be drawn to closely match the post mortem slice sections used in the in vitro analysis. In one aspect the parameters $K_1$, $k_2$, $k_3$, $k_4$, and BP can be measured by least squares fitting of PET. However, other suitable measurement techniques can be used.

Automated image display software has been developed for this work, and can be used to orient the myocardium to the standard short- and long-axis display as well as for generating polar maps by automated segmentation methods. Methods have been established for such automation. These methods can be important in the data analysis and validation of the measured parameters so that inter-observer variations can be eliminated.

*In vitro validation of MC-I with biochemical assays:*

Tissue samples each of normal, hibernating, and infarcted myocardium (200 mg/sample) can be isolated for MC-I Western blot analysis, to compute the sample mean and standard error of the mean. In one aspect, 20 tissue sample can be isolated to obtain statistically significant results. Pathology analysis of the central portion of each sample (e.g., 2x2 mm/sample) can also be performed. In brief for pathology analysis, the central portion of each sample can be preserved in, for example, 4% paraformaldehyde overnight, sectioned, and analyzed for confirmation of normal, hibernating, and infarcted tissue characteristics.

In some aspects, Western blot analysis can be a useful tool for assessing protein concentrations both quantitatively and qualitatively. For example, tissue samples (e.g., 200 mg) can be snap-frozen in liquid nitrogen, then homogenized in several (e.g., 9) volumes buffer at 4°C to create tissue lysates. Total protein can be extracted and quantitated to 15 $\mu$g protein per sample and treated with protease inhibitor cocktail tablets. The expression level of intact MC-I multimeric complex per 15 $\mu$g total protein can be analyzed by 4-12% 1-D SDS-PAGE (Novex), transferred to a PDVF membrane, non-specifically blocked, probed using primary mouse anti-bovine MC-I monoclonal antibody against the 8kDa subunit specific for MC-I (Mitosciences, MS109, 1:500 dilution in TBS, 0.1% Tween 20, 5% BSA) for 2 hrs at 25°C. However, any other suitable probe that binds to MC-I can be substituted or used in addition to the aforementioned probe. In one aspect, actin and porin can serve as mitochondrial housekeeping standards, and 4 $\mu$g of isolated bovine mitochondria (Mitosciences, MS802) can serve as the positive control. The membrane can be washed several times, incubated with secondary anti-mouse antibody conjugated to horseradish peroxidase (Amersham Biosciences) for 2 hrs at 25°C, washed, and developed using the ECL Western blot detection kit (Amersham Biosciences). Some primary anti-MC-I antibodies are reactive against bovine, human, mouse, and rat MC-I, and are also specific against pig MC-I.

The described approach, not forming part of the claimed invention nor the claimed embodiments, can allow quantitation and comparison of MC-I protein expression levels (i.e., the number of BMS tracer binding sites) in normal, hibernating, and infarcted myocardium. Based on previously reported work, a reduction of MC-I complex of 70% in infarcted myocardium and 20-40% in hibernating myocardium is anticipated.

The described methods can have two possible limitations. First, contaminating peripheral blood cannot be fully removed due to microsphere small vessel occlusion, it can be important to take into consideration differences in myocardial blood flow, which can be measured for each tissue sample used. Nevertheless, this is not a major limitation depending on the amount of microspheres used and can be less of a limitation for a trace amount of microspheres.

Second, if differential Western blot quantitation of MC-I complexes in each myocardial tissue type is unsuccessful, the relative protein expression of the 44 respiratory chain subunits and 3 assembly factors specific to MC-I in each myocardial tissue sample can be identified using protein mass spectrometry, for example, using an MC-I library.

*Quantitatively compare the difference of MBF and MC-I expression in three myocardium states (normal, ischemic, infarcted) and evaluate the performance of the proposed approach for a clinically relevant task:*

The time-activity curve of each segment can be fitted to the two compartment model described herein, in which the input function is extracted from generalized factor analysis of dynamic sequences (GFADS) and corrected for plasma concentration. The parameters $K_1$ and BP can be computed for each segment of the myocardium. From the PET data, there can be about 25 values of $K_1$ and BP for each of the subjects in question (e.g., about 200 values for 8 pigs). From in vitro analysis of microsphere and MC-I activity, there can be a corresponding about 25 values of MBF and about 25 values of MC-I expression level per subject/pig. Several analyses can be performed with an increasing level of complexity and clinical significance.

One possible analysis, not forming part of the claimed invention nor part of the claimed embodiments of the invention, is the within method. Here, segment condition can be classified as normal, ischemic, or infracted according to visual inspection of the tetrazolium-stained myocardium. The in vitro MC-I and blood flow assays can be compared separately. The statistical analysis can follow a block design, with the subject (pig) representing the block and condition the categorical variable. It is anticipated that normal, ischemic and infracted values can be significantly different. A similar analysis can be done for PET blood flow and BP.

A second possible analysis is a pooled analysis. In this analysis the in vitro end points can be plotted versus the PET endpoints. For example, considering Western blot estimates of MC-I expression level and PET BP as corresponding variables, each segment in each subject (pig) contributes a measurement of MC-I expression level. The plot can, in one example, contain approximately 200 points. By pooling the data, the classification into condition becomes unimportant. Nevertheless, the data can be identified by subject (pig) allowing for differences attributable to the individual sample (pig) to be taken into account. Since both the PET and in vitro estimates have noise, it may not be possible to use simple regression techniques; instead, a total least squares approach can be used. Despite these caveats, the plot can reveal a correlation. In one possible outcome, dynamic $^{18}$F-Flurpiridaz PET MC-I and MBF computed for all myocardial segments are strongly and positively (r > 0.8) correlated with measures obtained using in vitro assay and microspheres by correlation analysis. For N=200, it can be desirable to achieve a statistical power of 89% for $\alpha$=0.05.

A third possible analysis method involves discriminant analysis. Discriminant analysis can be performed to determine, in examples not forming part of the claimed invention nor part of the claimed embodiments, the discriminant power that can be achieved in discriminating among normal, hibernating and infracted myocardium using either MC-I, or MBF variables as well as a vector of two variables, namely the MC-I binding potential and the MBF as measured by PET, such as El Fakhri G, Kijewski MF, Albert MS, Johnson KA, Moore SC. Quantitative SPECT leads to improved performance in discrimination tasks related to prodromal Alzheimer's disease. J Nucl Med. Dec 2004;45(12):2026-2031. A comparison can be made for the discriminant power achieved with PET and the reference discriminant power that can be achieved using the in-vivo MC-I assay and microsphere measurements.

## EXAMPLE 3

*Preliminary study in a primate model with $^{18}$F-Flurpiridaz:*

Based on preliminary data from a primate study using $^{18}$F-Flurpiridaz, the parameter precision for estimating the MBF and $BP_P$ was evaluated. Under approved IACUC protocol, a 100 minute dynamic $^{18}$F-Flurpiridaz study (1.25 mCi) was conducted on a 4.1-kg cynomolgus monkey, using a microPET P4.

The microPET Primate 4-ring system (P4) is an animal PET tomograph with a 7.8 cm axial extent, a 19 cm diameter transaxial field of view (FOV) and a 22 cm animal port. The system is composed of 168 detector modules, each with an 8 x 8 array of 2.2 x 2.2 x 10 mm3 lutetium oxyorthosilicate crystals, arranged as 32 crystal rings 26 cm in diameter. The detector crystals are coupled to a Hamamatsu R5900-C8 PS-PMT via a 10 cm long optical fibre bundle. The detectors have a timing resolution of 3.2 ns, an average energy resolution of 26%, and an average intrinsic spatial resolution of 1.75 mm. The system operates in 3D mode without inter-plane septa, acquiring data in list mode. The reconstructed image spatial resolution ranges from 1.8 mm at the centre to 3 mm at 4 cm radial offset. The tomograph has a peak system sensitivity of 2.25% at the centre of the FOV with a 250-750 keV energy window. The noise equivalent count rate peaks at 100-290 kcps for representative object sizes.

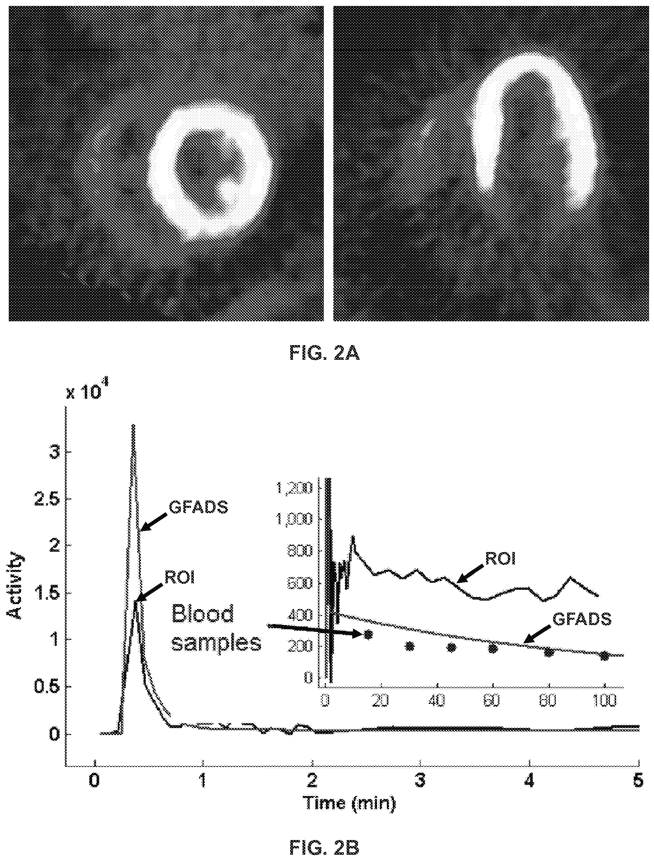

Fig. 2A shows the summed images of the myocardium in the short and long axis. The left ventricle (LV) and right ventricle (RV) input functions were extracted with GFADS. Fig. 2B shows the GFADS-extracted LV input function compared to the blood samples and ROI-based input function. The GFADS-input function aligns well with the GFADS data and has less partial volume effect and less contamination from spillover.

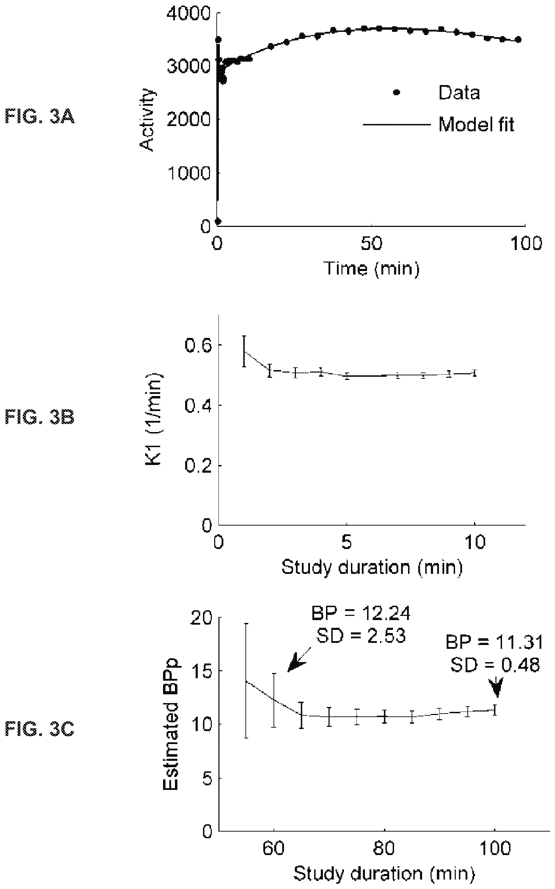

Time-activity curve from a region of interest drawn over the myocardium was fitted to the 2-compartment model. Parameter SD is computed from the asymptotic covariance matrix derived from the non-linear least squares fitting. The estimated parameters are: $K_1$ = 0.51 (SD=1.5%), $k_2$ = 0.075 (SD =4.7%), $k_3$ = 0.026 (SD=10.2%), $k_4$ = 0.016 (SD=10.4%), $k_3/k_4$ = 1.65 (SD=4.0%). Fig. 3A shows the fitted time-activity curve. To evaluate the precision of $K_1$ and $BP_P$, different study durations were used ranging from one minute to 100 minutes to estimate $K_1$ and BP as plotted for $K_1$ in Fig. 3B and for $BP_P$ in Fig. 3C. It was found that, with 10 minutes of data, estimate of $K_1$ is able to achieve a high precision (SD<10%). On the other hand, BP can require a longer measurement period.

The model selection has also been tested by quantitatively evaluating the goodness of fit with different model configurations. The 2-compartment, reversible model has the a number of parameters (six, including spillover fractions from LV and RV) to estimate but can reduce the residual as compared to the 2-compartment, irreversible model (five parameters to estimate with $k_4$ neglected) and the 1-compartment model (no binding, four parameters to estimate). The Akaike Information Criterion (AIC) and F-test can be used to objectively and quantitatively determine whether a more complex model is preferred and significantly reduce the residual. Results are summarized in Table 1.

**TABLE 1:** Comparison of the goodness of fit using 3 model configurations.

|  | RMSE | F-ratio | AIC ratio |
|---|---|---|---|
| 2-compartment reversible model | 1.12 |  |  |
| 2-compartment irreversible model | 1.78 | 28.29* | 0.83 |
| 1-comparment model | 5.41 | 22.96** | 0.58 |

In Table 1, RMSE is calculated through weighted least squares with weights approximated by data variance. F-ratio is calculated from the F-test between the 2-comp reversible model vs. the other two models. Critical values are 4.06 (*) and 3.2 (**) respectively ($\alpha$=0.05). When F-ratio is greater than the critical value, the more complex model is preferred and significantly reduces the residual. Akaike Information Criterion (AIC) ratios are calculated from the ratio between the AIC from 2-comp reversible model and the AICs from the other two models. An AIC ratio that is lower than one indicates that the more complex model is the preferred model.

It was found that, the 2-compartment reversible model has the lowest root mean square error (RMSE), significantly reduces the residual in the F-test and AIC. All three criterions confirm that, when fitting the data from the primate study, the 2-compartment model with binding mechanisms is preferable, indicating that the binding is a component that can be important to take into account when the study duration is relatively long.

## EXAMPLE 4

*Preliminary analysis for MBF in clinical studies with $^{18}$F-Flurpiridaz:*

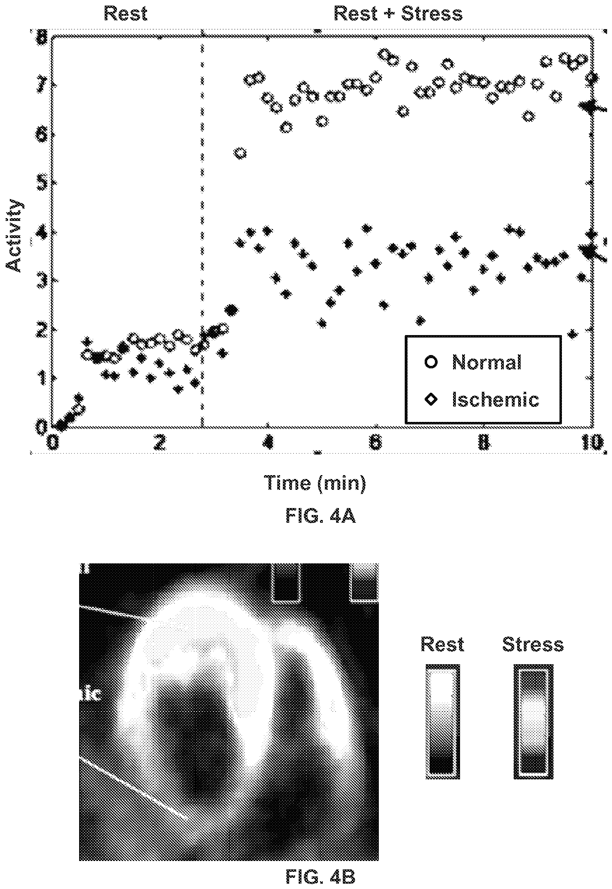

MBF was computed with kinetic modeling analysis in a group of subjects in phase II clinical trial of $^{18}$F-Flurpiridaz. Rest and stress PET studies were performed separately on 10 healthy subjects and 6 CAD patients (Figs. 4A-4B). In the clinical trial, imaging was terminated after 30 minutes and determination of BP was not feasible. MBF was estimated for each subject over a viable myocardial region and, if applicable, an ischemic region that showed mismatch between the rest and stress studies. The estimated MBF for rest-stress and healthy/ischemic myocardium are summarized in **Table 2.**

TABLE 2: MBF (mL/g/min) estimated in clinical $^{18}$F-Flurpiridaz studies.

|  | Healthy Subjects | Normal Myocardium in Patients | Ischemic Myocardium |
|---|---|---|---|
| Rest | 0.67±0.01 | 0.73±0.04 | 0.86±0.06 |
| Stress | 2.84±0.06 | 1.78±0.07 | 0.73±0.07 |

During the rest study, there was no significant difference in MBF (t-test, $\alpha=0.05$) between the healthy and ischemic myocardium. However during pharmacological stress, MBF increased more than threefold in healthy myocardium and remained approximately the same in ischemic regions. The MBF measured with kinetic modeling for $^{18}$F-Flurpiridaz are consistent with the previously reported MBF. Due to the short acquisition duration of these studies, satisfactory precision of the estimated BP was not achieved. The kinetic analysis of the $^{18}$F-Flurpiridaz shows its high potential in absolute quantitation of MBF in clinical cardiac PET.

## EXAMPLE 5

*Preliminary simulation studies for measuring BP:*

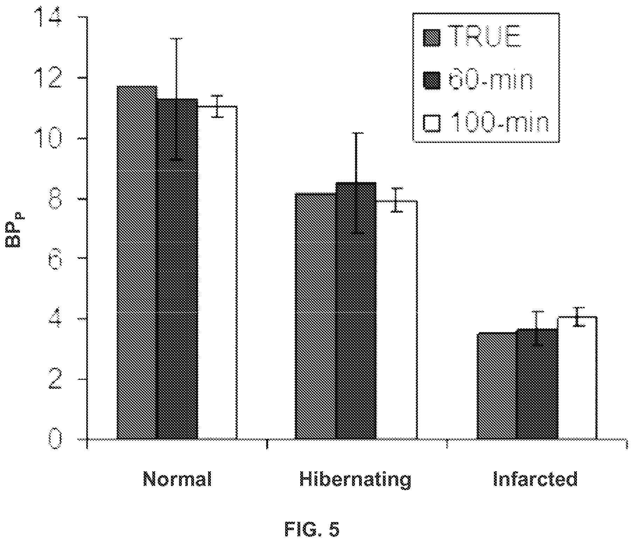

A simulation study was conducted to evaluate the parameter precision in different states of myocardium. Monte Carlo simulations were used to generated realistic dynamic PET data using time-activity curves simulated with $BP_P$ = 11.3 for normal myocardium, 8.2 for hibernating (30% reduction) and 3.5 for infarcted (70% reduction). 50 noise realizations were repeated by adding the Poisson deviates to the simulated noise-free sinogram data followed by reconstruction. Using data with 60 minutes and 100 minutes produced similar bias (Fig. 5). The $BP_P$ is underestimated in the normal region (about -5%) and over-estimated in the infarcted region (about 10%). This difference can be attributed to the lower $k_3$ in the infarcted myocardium; thus accurate BPp estimation can require a longer study duration. In terms of precision, the 60-min fit has a SD of 17.1%, 20.4% and 15.9% in normal, hibernating and infarcted myocardium, while the 100-min SD is 3.0%, 4.7% and 7.9% respectively. These preliminary data show that the $BP_P$ estimated from the three ischemic states follows the same trend of precision.

In an aspect of the disclosed concepts, the product will be a technique -- based upon PET imaging with agents such as [$^{18}$F]-Flurpiridaz -- for mapping the regional distribution of mitochondria. As detailed in the application, literature searches have turned up a number of diseases where mitochondria dysfunction is implicated and for which this imaging technique would be applicable for research or clinical care.

In one aspect of the disclosure, methods for quantitating MC-I expression level from a single $^{18}$F-Flurpiridaz PET study are provided. In one aspect, the sensitivity of computed MC-I binding potential can be examined as a function of study duration. Based on the sensitivity analysis, an experimental protocol is provided that is both clinically feasible and provides reliable quantitation of MC-I expression.

In another aspect of the disclosure, a chronic porcine model of myocardial ischemia with different degrees of severity can be used to alter the MC-I expression as well as MBF. The MC-I expression level and MBF measured with $^{18}$F-Flurpiridaz PET can, in an example not forming part of the claimed invention nor claimed embodiments, be compared to reference in vitro assays and microspheres, respectively. In one aspect, chronic ischemia is induced in the porcine myocardium with partial and complete occlusion in the LAD and LCX coronary territories to alter the MC-I expression levels. MC-I expression levels can be quantified by $^{18}$F-Flurpiridaz PET and in vitro analysis. In another aspect, not forming part of the claimed invention nor claimed embodiments, the computed MC-I expression level and MBF from $^{18}$F-Flurpiridaz PET kinetic parameters can be compared against reference laboratory methods for in vitro assays for MC-I expression and microspheres (MBF).

In yet another aspect, not forming part of the claimed invention nor claimed embodiments, the difference of MBF and MC-I expression can be compared in three myocardium states (normal, ischemic, infarcted) to evaluate the performance of the proposed approach for the clinical task of discriminating between the three states. The accuracy and precision of MBF and MC-I expression computed with $^{18}$F-Flurpiridaz dynamic PET is assessed as compared to reference in vitro assays and microspheres.

## EXAMPLE 6

*$^{18}$F-Flurpiridaz cardiac PET study in infarcted pig:*

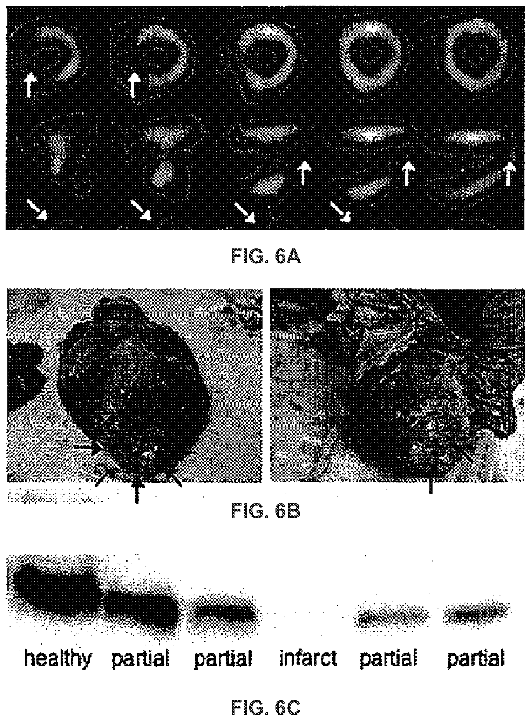

Two domestic swine were studied using procedures similar to those described herein. Each animal underwent surgical infarction and dynamic PET imaging with $^{18}$F-Flurpiridaz, which was radiolabeled using synthesized precursors. Reconstructed PET images showed clear infarction of the myocardium, such as the apical-anteroseptal lesion evident in FIG.6A, which was corroborated at dissection (see photograph in FIG. 6B). Western blots revealed decreased MC-I expression in damaged tissue samples as compared to healthy myocardium (FIG. 6C). GFADS was applied to the dynamic PET series to extract curves from the left and right ventricle. As observed in the preliminary monkey study, the LV curve extracted by GFADS was in good agreement with the arterial blood samples. PET concentration curves were extracted from dynamic PET data in regions of interest delineating the 17 standard segments of the heart.

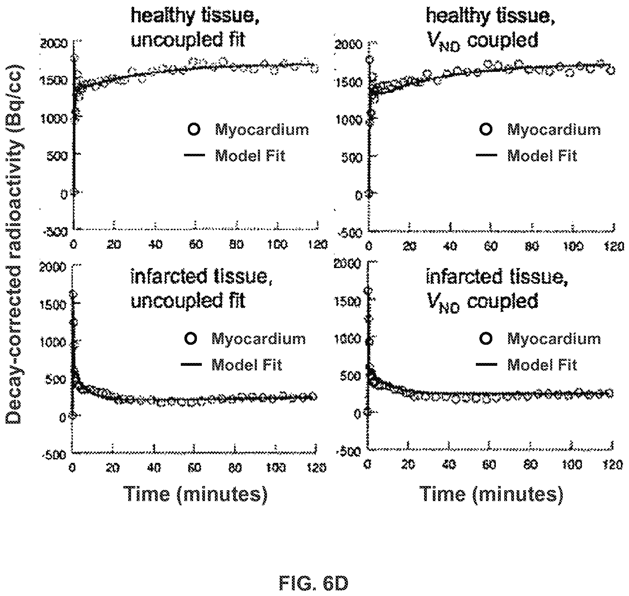

The tissue curves were analyzed using the two-tissue compartment model with reversible binding as described above. Again similar to the monkey study, this model fit the data from healthy myocardium very well; moreover, fits in damaged tissues were also very good (FIG. 6D). Fitting the 17 segments independently yielded unreliable estimates BP, but values for $K_1$ and the macroparameter $V_T$ were robust (typical COV < 10% across regions) and markedly reduced in damaged compared to healthy tissue. In damaged tissues, reductions of $V_T$ (about 75%) were greater than those of K1 (about 50%); suggesting that decreases in $V_T$ could not be fully explained by lower perfusion: therefore, reduced binding of $^{18}$F-Flurpiridaz at MC-I is implicated.

Using the two-tissue model with $k_4$ coupled across regions improved the precision of the individual rate constants as well as the macroparameters: $K_1$, $V_T$, and *BP* were reliably estimated in most regions, although outlier *BP* values remained. The global value of $k_4$ was consistent with the distribution observed in uncoupled fits. $K_1$, $V_T$, and *BP* were all lower in damaged tissues than healthy; $V_T$ was again lowered more than $K_1$, consistent with the reduced specific binding now apparent due to more precise estimation of *BP*. The model fits to data were very good, with almost imperceptible effect on the goodness of fit in most regions as compared to the uncoupled fits.

A grand fit that coupled $V_{ND}=K_1/k_2$ across regions produced a general effect similar to the use of a common *k4* but with somewhat greater influence on quality of fit, especially in ischemic and infracted segments (FIG. 6D). The global $V_{ND}$ was representative of values obtained from uncoupled fits. Notably, fitting a common $V_{ND}$ stabilized *BP* estimates even more than coupling *k4; BP* values were robust and indicated binding reductions of up to 90% in damaged tissues. Combining the grand fits to couple both $V_{ND}$ and *k4* produced model fits that were slightly worse than those from uncoupled fits or grand fits on one parameter, but were still satisfactory. The precision of estimated parameters was further improved over the beneficial effects seen when coupling a single common parameter and the differentiations between regional $K_1$, $V_T$, and *BP* values in healthy, ischemic, and infarcted tissue were retained. The parameter variance reduction owing to the use of coupled parameters was even greater when shorter scan durations (e.g., 60 minutes of data as compared to 120) were evaluated. This observation presents parameter coupling as a promising strategy in the design of an optimal and practical experimental protocol. These results demonstrate (i) the ability to execute the porcine model of ischemia, (ii) Western blotting for quantification of MC-I expression, and (iii) that the two-tissue compartment model - particularly for grand fits using common parameters across regions - provides information about regional perfusion and MC-I binding in healthy and damaged tissue from a single dynamic PET study.

In summary, a PET measurement of MC-I receptor density can be considered an in vivo assay of MC-I density. MC-I density is affected by a number of diseases, both chronic and acute. As an in vivo assay, there are a number of components required, including an analytic procedure, involving software, to estimate the density. The components include use of an appropriate radiopharmaceutical; flurpiradaz is an expedient example but not ideal, due to its slow kinetics. There is also a laboratory element in which the patient is scanned in a manner appropriate to the assay, not a single image. Various examples of the disclosure encompass all of these components and can allow non-invasive quantitation of mitochondrial activity.

Each reference identified in the present application is herein incorporated by reference in its entirety.

The foregoing description is meant to be exemplary, and does not limit the scope of present disclosed concepts.

**Claims**

1. A method for in vivo assay and imaging the expression of mitochondrial complex I (MC-I), the method comprising:

imaging a living subject to whom a PET ligand $^{18}$F-Flurpiridaz, which binds reversibly to MC-I in the tissues of the subject, has been administered, with a positron emission tomography (PET) imaging system and storing at least one PET image of the subject in a non-transitory computer readable media;
fitting regional time-activity curves to a two-tissue compartment model using a computer processor adapted to calculate MC-I expression levels based on a kinetic model stored within non-transitory computer readable media, wherein the processor is adapted to provide quantitative mapping of regional MC-I expression, the MC-I expression levels being based on said PET imageable binding reaction between the PET ligand and MC-I.

2. The method of claim 1 further comprising processing images with a computer processor that calculates a level of blood flow (BF) within the subject by analyzing portions of the images of the subject expressing levels of the presence of the PET ligand.

3. The method of claim 2, wherein the calculated levels of BF are calculated based on a kinetic model stored within computer readable media.

4. The method of claim 1, wherein the method further comprises identifying states of at least one of a metabolic disorder, neurological disorder, neuromuscular disorder, and psychiatric disorder based on the identified interaction between a PET ligand and MC-I.

5. The method of claim 2, wherein the BF comprises myocardial blood flow (MBF).


**Patentansprüche**

1. Verfahren für In-vivo-Test und Bildgebung der Expression von Mitochondrial Complex I (MC-I), wobei das Verfahren Folgendes umfasst:

Bildgebung bei einem lebenden Individuum, dem ein PET-Ligand $^{18}$F-Flurpiridaz verabreicht wurde, der an MC-I in den Geweben des Individuums reversibel bindet, mit einem Positronenemissionstomographie(PET)-Bildgebungssystem und Speichern wenigstens eines PET-Bilds des Individuums auf einem nicht transitorischen Datenträger;
Anpassen regionaler Zeit-Aktivität-Kurven an ein Zwei-Gewebekompartimente-Modell unter Verwendung eines Computerprozessors, der zur Berechnung von MC-I-Expressionsniveaus anhand eines in nicht transitorischen Datenträgern gespeicherten kinetischen Modells adaptiert ist, wobei der Prozessor so adaptiert ist, dass eine quantitative Kartierung regionaler MC-I-Expression geliefert wird, wobei die MC-I-Expressionsniveaus auf der einer PET-Bildgebung zugänglichen Bindungsreaktion zwischen dem PET-Liganden und MC-I beruhen.

2. Verfahren nach Anspruch 1, ferner umfassend Prozessieren von Bildern mit einem Computerprozessor, wobei ein Niveau von Blutfluss (BF) im Individuum durch Analysieren von Teilen der Bilder des Individuums mit Expressionsniveaus des Vorliegens des PET-Liganden berechnet wird.

3. Verfahren nach Anspruch 2, wobei die berechneten BF-Niveaus anhand eines in Datenträgern gespeicherten kinetischen Modells berechnet werden.

4. Verfahren nach Anspruch 1, wobei das Verfahren ferner Identifizieren von Zuständen wenigstens einer von einer Stoffwechselstörung, neurologischen Störung, neuromuskulären Störung und psychiatrischen Störung anhand der identifizierten Wechselwirkung zwischen einem PET-Liganden und MC-I umfasst.

5. Verfahren nach Anspruch 2, wobei der BF Myokard-Blutfluss (MBF) umfasst.

**Revendications**

1. Procédé de dosage et d'imagerie *in vivo* de l'expression du complexe mitochondrial I (MC-I), le procédé comprenant :

   l'imagerie d'un sujet vivant auquel un ligand de TEP $^{18}$F-Flurpiridaz, qui se lie de façon réversible à MC-I dans les tissus du sujet, a été administré, avec un système d'imagerie par tomographie par émission de positrons (TEP) et le stockage d'au moins une image TEP du sujet dans un support lisible par ordinateur non transitoire ;
   l'ajustement de courbes temps-activité régionales à un modèle à deux compartiments tissulaires au moyen d'un processeur informatique adapté pour calculer les taux d'expression de MC-I sur la base d'un modèle cinétique stocké dans un support lisible par ordinateur non transitoire,
   dans lequel le processeur est adapté pour produire une cartographie quantitative de l'expression régionale de MC-I, les taux d'expression de MC-I étant basés sur ladite réaction de liaison pouvant être imagée par TEP entre le ligand de TEP et MC-I.

2. Procédé selon la revendication 1 comprenant en outre le traitement d'images avec un processeur informatique qui calcule un débit de sang (BF) dans le sujet par analyse de parties des images du sujet exprimant des taux de la présence du ligand de TEP.

3. Procédé selon la revendication 2, dans lequel les taux calculés de BF sont calculés sur la base d'un modèle cinétique stocké dans le support lisible par ordinateur.

4. Procédé selon la revendication 1, dans lequel le procédé comprend en outre l'identification d'états d'au moins l'un parmi un trouble métabolique, un trouble neurologique, un trouble neuromusculaire et un trouble psychiatrique sur la base de l'interaction identifiée entre un ligand de TEP et MC-I.

5. Procédé selon la revendication 2, dans lequel le BF comprend le débit sanguin myocardique (MBF).

$$C_p \xrightarrow{K_1} \xleftarrow{k_2} C_F \xrightarrow{k_3} \xleftarrow{k_4} C_B$$

FIG. 1A

| Blood Sampling | PET Data Acquisition And Reconstruction |
|---|---|
| Measured or Extracted Input Function | Definition of Region of Interest (ROI) |

$$C_p(t) \qquad C_F(t) + C_B(t)$$

Non-linear least square (NLLS) Curve fitting

## Estimation of K1~k4 and BP

FIG. 1B

FIG. 1C

FIG. 2A

FIG. 2B

FIG. 3A

FIG. 3B

FIG. 3C

FIG. 4A

FIG. 4B

FIG. 5

FIG. 6A

FIG. 6B

healthy partial partial infarct partial partial

FIG. 6C

FIG. 6D

FIG. 7

200

202 → administer MC-I ligand

204 → image subject

206 → store image(s)

208 → analyze image(s)

210 → calculate perfusion and/or MC-I expression

212 → diagnose myocardial state

FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Advances in PET myocardial perfusion imaging: F-18 labeled tracers. **C. RISCHPLER et al.** Annals of Nuclear Medicine. Springer, 09 November 2011, vol. 26, 1-6
- **PAGE B ; YOUNG R ; IYER V et al.** Persistent regional downregulation in mitochondrial enzymes and upregulation of stress proteins in swine with chronic hibernating myocardium. *Circ Res.,* 04 January 2008, vol. 102 (1), 103-112
- **LOGAN J ; FOWLER JS ; VOLKOW ND et al.** Graphical analysis of reversible radioligand binding from time-activity measurements applied to [N-11C-methyl]-(-)-cocaine PET studies in human subjects. *J Cereb Blood Flow Metab,* September 1990, vol. 10 (5), 740-747
- **ZHOU Y ; YE W ; BRASIC JR ; CRABB AH ; HILTON J ; WONG DF.** A consistent and efficient graphical analysis method to improve the quantification of reversible tracer binding in radioligand receptor dynamic PET studies. *Neuroimage,* 01 February 2009, vol. 44 (3), 661-670

- **WU et al.** *The Journal of Nuclear Medicine,* 1998, vol. 39, 117-425
- **EI FAKHRI G ; KARDAN A ; SITEK A et al.** Reproducibility and accuracy of quantitative myocardial blood flow assessment with (82)Rb PET: comparison with (13)N-ammonia PET. *J Nucl Med.,* July 2009, vol. 50 (7), 1062-1071
- **EI FAKHRI G ; SITEK A ; GUERIN B ; KIJEWSKI MF ; DI CARLI MF ; MOORE SC.** Quantitative dynamic cardiac 82Rb PET using generalized factor and compartment analyses. *J Nucl Med.,* August 2005, vol. 46 (8), 1264-1271
- **EI FAKHRI G ; KIJEWSKI MF ; ALBERT MS ; JOHNSON KA ; MOORE SC.** Quantitative SPECT leads to improved performance in discrimination tasks related to prodromal Alzheimer's disease. *J Nucl Med.,* December 2004, vol. 45 (12), 2026-2031